(19) Europäisches Patentamt

European Patent Office

Office européen des brevets

(11) Publication number: **0 431 323 A1**

## (12) EUROPEAN PATENT APPLICATION

(21) Application number: 90121149.0

(22) Date of filing: 05.11.90

(51) Int. Cl.⁵: **C12P 1/06**, C12N 1/20, C07G 11/00, A61K 35/66, //(C12P1/06,C12R1:03), (C12N1/20,C12R1:03)

(30) Priority: 06.11.89 US 431811

(43) Date of publication of application:
12.06.91 Bulletin 91/24

(84) Designated Contracting States:
AT BE CH DE DK ES FR GB GR IT LI LU NL SE

(71) Applicant: **Bristol-Myers Squibb Company**
**345 Park Avenue**
**New York, N.Y. 10154(US)**

(72) Inventor: **Sugawara, Koko**
**2-1-14-102, Minami**
**Wako-shi, Saitama(JP)**
Inventor: **Nishiyama, Yoji**
**2-15-7-301 Kuramac**
**Taitoh-ku, Tokyo(JP)**
Inventor: **Yamamoto, Haruaki**
**702-3, Uchinomiya**
**Tama-shi, Tokyo(JP)**

(74) Representative: **Kinzebach, Werner, Dr. et al**
**Patentanwälte Reitstötter, Kinzebach und**
**Partner Sternwartstrasse 4 Postfach 86 06 49**
**W-8000 München 86(DE)**

(54) Antitumor antibiotic BU-3983T.

(57) The invention relates to a new antitumor antibiotic compound BU-3983T which is produced by cultivating a BU-3983T-producing strain of Actinomadura verrucosospora. BU-3983T exhibits antitumor activity.

EP 0 431 323 A1

## ANTITUMOR ANTIBIOTIC BU-3983T

## BACKGROUND OF THE INVENTION

### 1. Field of the Invention

The present invention relates to a novel antitumor antibiotic, BU3983T, and a process for preparing same by culturing microorganisms belonging to the genus Actinomadura. This compound demonstrates a high level of antitumor activity against B16 melanoma.

### 2. Description of the Prior Art

Amongst the prior art references of which the applicants are aware, the following represent what is believed to be the closest prior art.

Sheehan et al, J. Am. Chem. Soc., 90, 462 (1968), discloses the structure of the antibiotic Telomycin. It is a peptide lactone that has eleven amino acids and a molecular weight of 1272. It is active against Gram-positive organism, but has little or no effect against most Gram-negative bacteria. Telomycin was isolated from the culture broth of an unidentified Streptomyces.

Maehr, et al, J. Antibiotics, 39, 17 (1986), discloses the antibiotic azinothricin. It contains a 19-membered cyclodepsipeptide ring composed of six amino acids and a side chain. It is primarily active against Gram-positive microorganisms. Azinothricin was isolated from the culture filtrate of Streptomyces sp.

Fujioka, et al, J. Org. Chem. 53, 2820 (1988), discloses the structure of FR900359. FR900359 is a cyclic depsipeptide composed of ten acid units and at most seven amino acids, and has a molecular weight of 1001. It shows inhibition of platelet aggregation and decrease of blood pressure. FR900359 was isolated from Ardisia crenata sims.

None of these references teaches the novel compound of the present invention or its use as an antitumor antibiotic agent.

## SUMMARY OF THE INVENTION

An Actinomadura, Strain No. Q886-2, was isolated from a soil sample collected in Batangas, Luzon Island, Philippines. A new antitumor antibiotic BU-3983T was obtained by fermentation of this strain. BU-3983T possesses antitumor activity against mouse B16 melanoma.

## DESCRIPTION OF THE DRAWINGS

Fig. 1 shows the HPLC profile of BU-3983T.
Fig. 2 shows the IR spectrum of BU-3983T.
Fig. 3 shows the $^1$H-NMR spectrum of BU-3983T in $CDCl_3$
Fig. 4 shows the $^{13}$C-NMR spectrum of BU-3983T in $CDCl_3$
Fig. 5 shows the degradation products of BU-3983T

## DETAILED DESCRIPTION OF THE INVENTION

An Actinomadura, Strain No. Q886-2, was collected from a soil sample of the Philippines. Based upon the morphology, cultural and physiological characteristics and cell chemistry, the above-mentioned strain was identified as a species of Actinomadura, i.e., Actinomadura verrucosospora. Actinomadura verrucosospora, Strain No. Q886-2 was found to produce a new antibiotic which showed specific activity against mouse B16 melanoma. This antibiotic designated BU-3983T was recovered from the fermentation broth with n-butanol and purified by a series of chromatographies. Physico-chemical properties and degradation results of BU3983T suggested a novel peptide having three amino acids, glycine, sarcosine, β-hydroxyleucine and a lipophilic fragment. The structure has not yet been determined. BU-3983T demonstrated potent cytotoxicity against various tumor cells but weak antimicrobial activity. It exhibited significant prolongation of life span in mice transplanted with B16 melanoma.

Morphology: The substrate mycelia are well-branched and non-fragmentary. Aerial mycelium is poorly formed. The aerial mycelia bear monopodially hook-shaped spore chains which contain 3 to 10 spores per chain. The spore are oval (0.6 - 1.0 x 1.0 - 1.5 $\mu$m) and have mostly warty surface.

Cultural and physiological characteristics: The growth is moderate on most descriptive media. The color of aerial mycelia is uniformly white. No pigments are produced.

Growth temperature ranges from 18°C to 50°C. No growth occurs at 53°C. Acid is formed from adonitol, L-arabinose, cellobiose, D-glucose, glycerol, D-mannitol, L-rhamnose, trehalose and D-xylose, while no acid is formed from D-arabinose, cellulose, dulcitol, erythritol, inositol, lactose, D-melezitose, melibiose, methyl-$\alpha$-glucoside, raffinose, D-sorbitol and L-sorbose.

Chemotaxonomy: Whole cell and purified cell wall contain meso-2,6-diaminopimelic acid. Whole cell contains glucose and madurose. Thus, Strain Q886-2 is placed in cell wall Type III$_B$. The phospholipids contain phosphatidylinositol but not nitrogenous phospholipids, and hence the strain belongs to Type P-I. Menaquinone contains MK-9(H$_8$) and minor Mk-9(H$_{10}$).

Taxonomic position: The morphology and chemotaxonomy of strain Q886-2 indicate that strain Q886-2 is similar to Actinomadura Lechevalier and Lechevalier 1970. In addition to the spore and spore-chain morphology, strain Q886-2 is closely related physiologically to Actinomadura madurae, and more closely to A. verrucosospora. Thus, strain Q886-2 was designated Actinomadura verrucosospora.

Table 1

| Cultural characteristics of strain Q886-2 | | | | |
|---|---|---|---|---|
| Medium | Growth | Aerial mycelium | Substrate mycelium | Diffusible pigment |
| Sucrose-nitrate agar (Czapek-Dox agar) | Poor | None | Colorless | None |
| Tryptone-yeast extract broth (ISP No. 1) | Moderate; floccose, not turbid | None | Colorless | None |
| Yeast extract-malt extract agar (ISP No. 2) | Moderate | Moderate; white (263) | Colorless | None |
| Oatmeal agar (ISP No. 3) | Poor | Poor; white (263) | Colorless | None |
| Inorganic salts-starch agar (ISP No. 4) | Moderate | Poor; white (263) | Pale Yellow (89) | None |
| Glycerol-asparagine agar (ISP No. 5) | Moderate | Very scant; white (263) | Yellowish white (92) | None |
| Peptone-yeast extract-iron agar (ISP No. 6) | Good | None | Pale yellow (89) | None |
| Tyrosine agar (ISP No. 7) | Moderate | Very scant; white (263) | Yellowish white (92) | None |
| Glucose-asparagine agar | Poor | None | Colorless | None |
| Nutrient agar | Moderate | None | Pale Yellow (89) | None |
| Bennett's agar | Moderate | Moderate; white (263) | Colorless | None |
| Observation after incubation at 28°C for 3 weeks. Color name, used: ISCC-NBS color-name charts | | | | |

Table 2.  Physiological characteristics of strain Q886-2

Decomposition of:

| | |
|---|---|
| Adenine | - |
| Casein | + |
| Hippuric acid | + |
| Hypoxanthine | + |
| Tyrosine | + |
| Xanthine | - |

Decarboxylation of:

| | |
|---|---|
| Benzoate | - |
| Citrate | - |
| Mucate | - |
| Succinate | - |
| Tartrate | - |

Production of:

| | |
|---|---|
| Amylase | + |
| Esculinase | + |
| Gelatinase | + |
| Nitrate reductase | + |
| Tyrosinase | - |
| Urease | - |

Growth in:

| | |
|---|---|
| Lysozyme, 0.01% | - |
| NaCl, 1% - 7% | + |
| "    9% | - |
| pH, 5.0 - 12 | + |
| 18°C - 50°C | + |
| 16°C and 53°C | - |

Utilization of and acid production from:

| | Utilization | Acid |
|---|---|---|
| Adonitol | NT | + |
| D(-)Arabinose | - | - |
| L(+)-Arabinose | + | + |
| Cellobiose | + | + |
| Cellulose | - | - |
| Dulcitol | - | - |
| Erythritol | NT | - |
| D-Fructose | + | NT |
| D-Galactose | + | NT |
| D-Glucose | + | + |
| Glycerol | + | + |
| Inositol | - | - |
| Lactose | - | - |
| D-Mannitol | + | + |
| D-Mannose | ± | - |
| D(+)-Melezitose | - | - |
| Melibiose | - | - |
| Methyl-α-glucoside | NT | - |
| Raffinose | - | - |
| L-Rhamnose | + | + |
| D-Ribose | - | NT |
| Salicin | +(w) | NT |
| Soluble starch | + | NT |
| D-Sorbitol | - | - |
| L-Sorbose | - | - |
| Sucrose | +(w) | NT |
| Trehalose | + | + |
| D-Xylose | +(w) | + |

+(w): weakly positive, ±: marginal, NT: not tested

A biologically pure culture of Actinomadura verrucosospora Strain No. Q886-2 has been deposited with the American Type Culture Collection, Rockville, Maryland, under the accession number ATCC-53905.

As is the case with other microorganisms, the characteristics of the new BU-3983T-producing culture of the present invention, Actinomadura verrucosospora ATCC-53905, are subject to variation. Recombinants, variants and mutants of the ATCC-53905 strain may be obtained by treatment with various known mutagens such as ultraviolet rays, X-rays, high frequency waves, radioactive rays and chemicals. Natural and induced variants, mutants and recombinants of Actinomadura verrucosospora ATCC-53905 which retain the characteristic of producing BU-3983T are intended to be encompassed by the present invention.

BU-3983T may be produced by cultivating a BU-3983T-producing strain of Actinomadura verrucosospora ATCC-53905 or a mutant or variant thereof, under submerged aerobic conditions in an aqueous nutrient medium. The organism is grown in a nutrient medium containing an assimilable carbon source, for example an assimilable carbohydrate. Examples of suitable carbon sources include lactose, glycerol, sucrose, corn starch, glucose, mannose, fructose, cellobiose, trehalose, mannitol and xylose. The nutrient medium should also contain an assimilable nitrogen source such as, for example, fish meal, peptone, soybean flour, peanut meal, cotton seed meal and corn steep liquor. Nutrient inorganic salts may also be incorporated in the medium and such salts may comprise any of the usual salts capable of providing sodium, potassium, ammonium, calcium, phosphate, sulfate, chloride, bromide, nitrate, carbonate or like ions.

Production of the BU-3983T antibiotic may be effected at any temperature conductive to satisfactory growth of the organism, i.e., approximately 18-50 degrees Celsius, (see Table 2) and is conveniently carried out at a temperature of about 28 degrees Celsius. Ordinarily, optimum production is obtained after incubation periods of about 3-6 days. The fermentation may be carried out in flasks and in laboratory or industrial fermentors of various capacities. When tank fermentation is to be carried out, it is desirable to produce a vegetative inoculum in a nutrient broth by inoculating the broth culture with a slant or soil culture or a lyophilized culture of the organism. After obtaining an active inoculum in this manner, it is transferred aseptically to the fermentation tank medium for large scale production of the antibiotic. The medium in which the vegetative inoculum is produced can be the same as, or different from, that utilized in the tank for the production of the new antibiotic as long as it is such that a good growth of the microorganism is obtained.

Production of the BU-3983T antibiotic can be followed during the fermentation by testing samples of the broth or extracts of the mycelial solids for antibiotic activity against organisms known to be sensitive to the antibiotic or by an in vitro cytotoxicity assay, e.g. using B16 melanoma cells.

When fermentation is complete, BU-3983T is recovered from the fermentation broth and separated by extraction with a suitable organic solvent followed by a series of column chromatographies. Examples 1 and 2 below illustrate specific procedures for obtaining BU-3983T in substantially purified form.

## Physico-chemical Properties

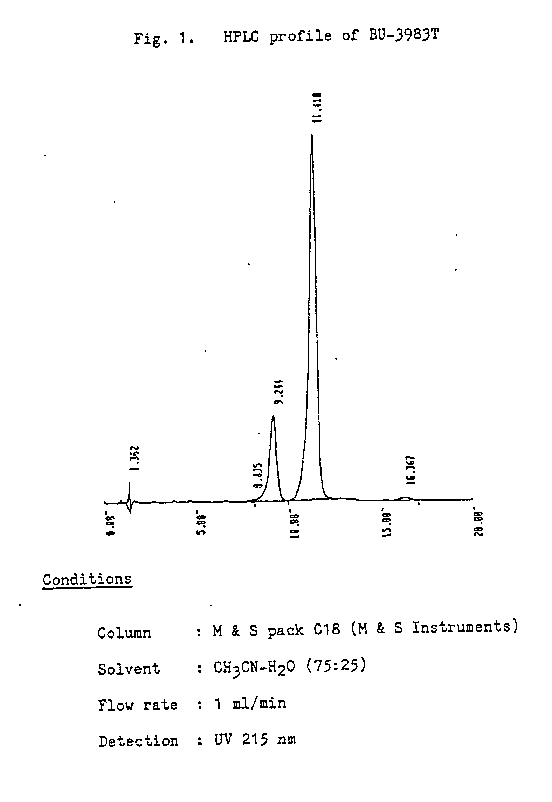

Physico-chemical properties of BU-3983T are summarized in Table 3. BU-3983T was obtained as a white amorphous powder which showed two peaks (Rt 9.2 and 11.4) in HPLC analysis as shown in Fig. 1. When these peaks were collected and reanalyzed by the same HPLC condition, they showed again two peaks (Rf 9.2 and 11.4) in a similar ratio (ca. 1:4). This result indicated that BU-3983T exists as a tautomeric mixture in solution.

This antibiotic is readily soluble in acetonitrile, methanol, chloroform, dimethyl sulfoxide and dimethylformamide, slightly soluble in n-butanol, ethanol and ethyl acetate, but practically insoluble in n-hexane and water. It gave a positive response to iodine vapor, AMS, and Rydon-Smith reagents. It was negative to ninhydrin, anthrone and Sakaguchi reaction.

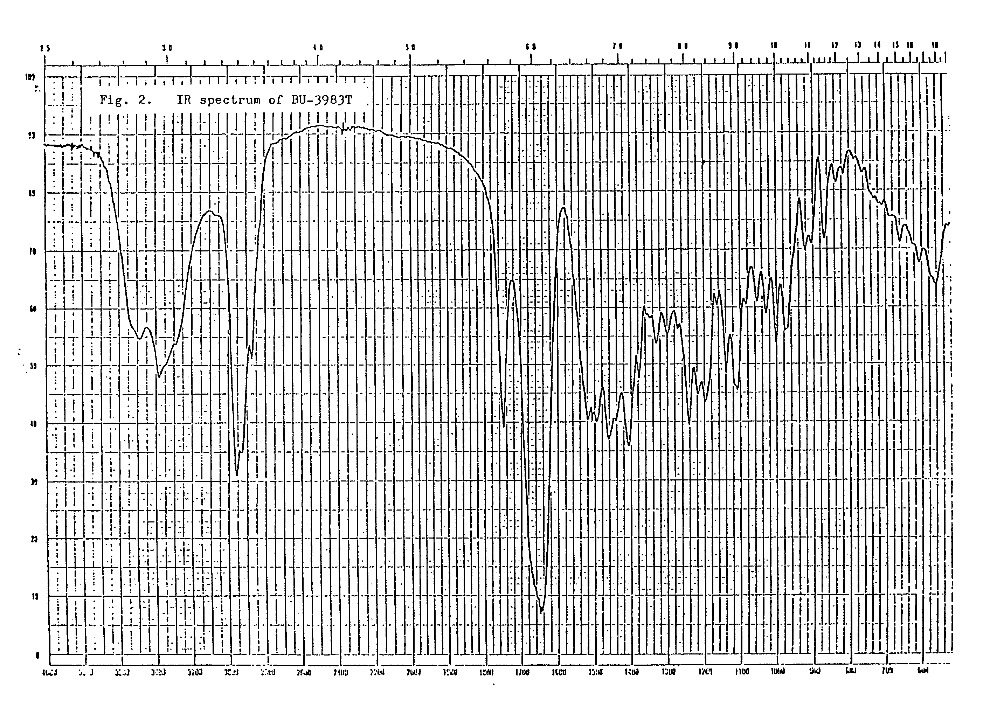

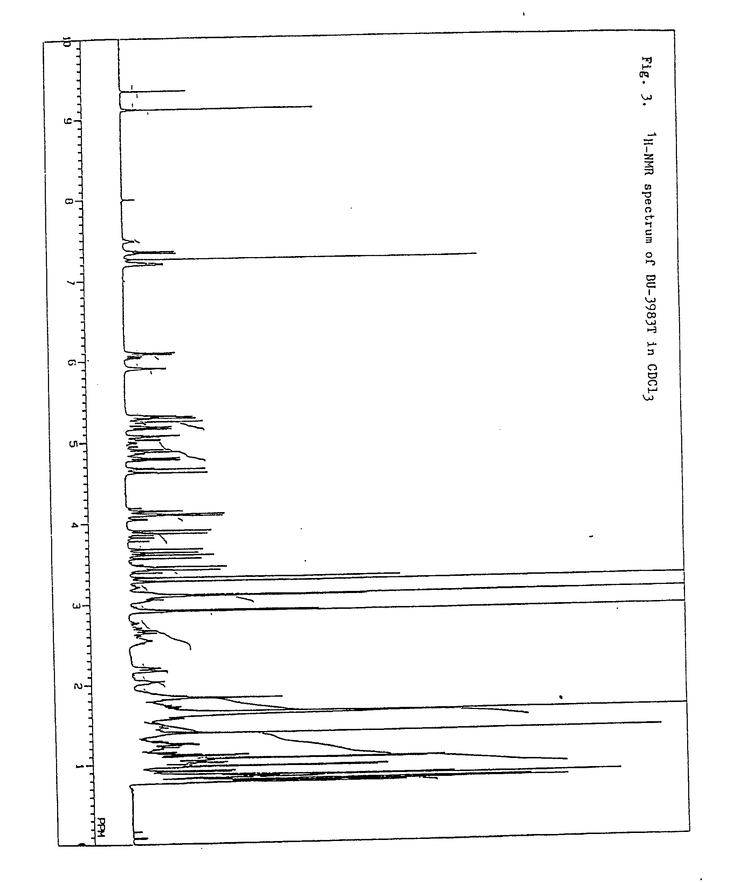

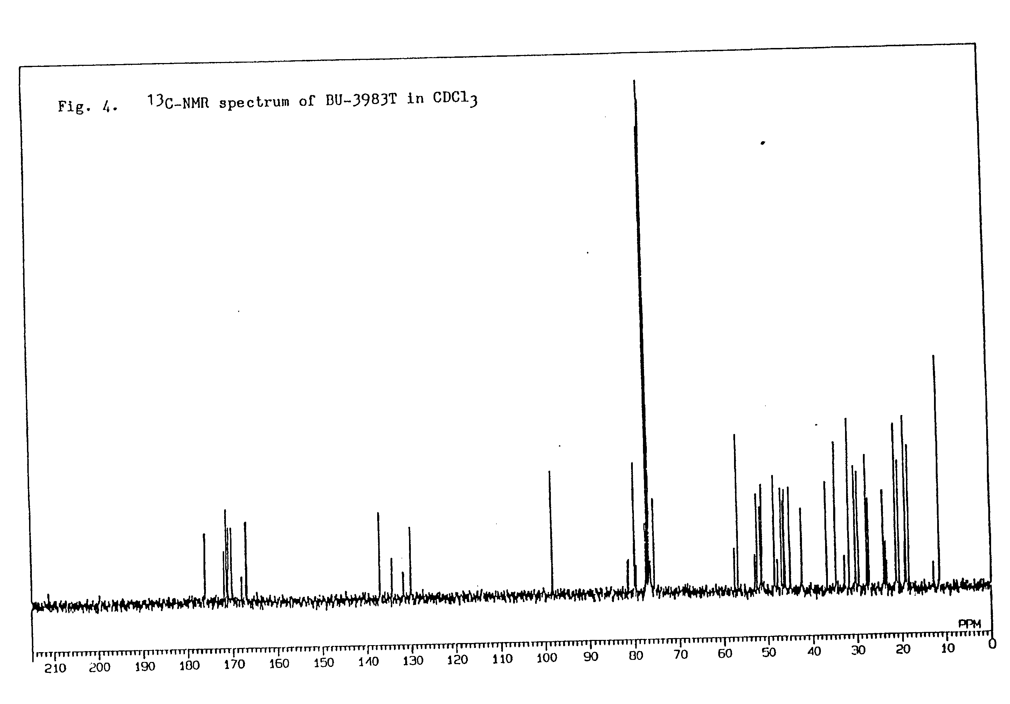

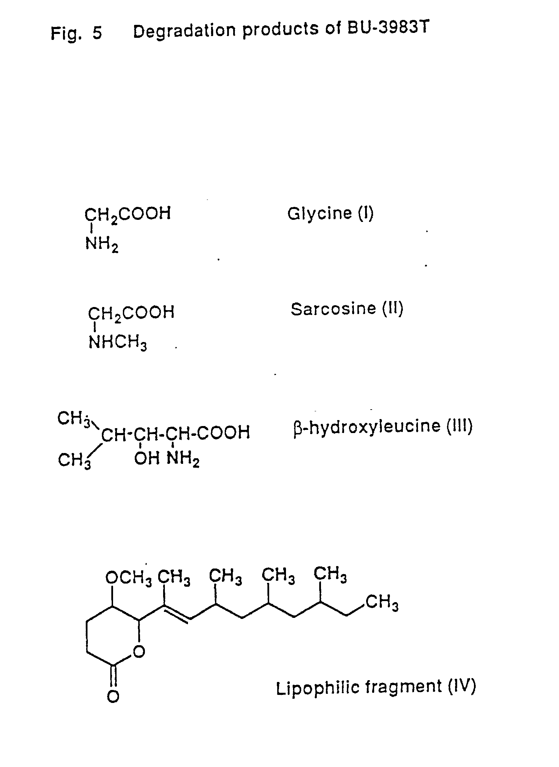

BU-3983T showed only end absorption in UV spectrum. The molecular formula of BU-3983T was determined to be $C_{43}H_{73}N_7O_{13}$ on the basis of the high resolution mass spectra (HR-FAB) and elemental analysis. The IR spectrum in KBr is shown in Fig. 2. A strong absorption band at 1640 $cm^{-1}$ was attributed to the amide carbonyl suggesting a peptide structure for the antibiotic. A band at 1750 $cm^{-1}$ which disappeared upon treatment of 0.1N sodium hydroxide indicated the presence of a lactone in its molecule. In the $^1$H-NMR spectrum (Fig. 3) and $^{13}$C-NMR (Fig. 4) spectra of BU-3983T, most of the signals were observed as double peaks with ca. 1:4 ratio indicating that the antibiotic was present as a tautomeric mixture in solution. Acid hydrolysis of BU-3983T with 6N hydrochloric acid produced three amino acids along with an unseparable mixture of lipophilic fragments. Two of them were determined as glycine (I) and sarcosine (II, 2 moles) by amino acid analysis and TLC behavior. The other amino acid III was isolated from the aqueous hydrolyzate of BU-3983T by Dowex 50W x 4 (Pyridine form) and Sephadex LH-20 column chromatographies, and determined to be $\beta$-hydroxyleucine on the basis of its SIMS spectrum (m/z 148 (M + H)$^+$) and $^1$H-and $^{13}$C-NMR spectral analyses. The structures of amino acids I, II and III are illustrated in Fig. 5.

When heated with 1N sodium hydroxide, BU-3983T gave a liphophilic fragment (IV) which was extracted from the hydrolyzate with ethyl ether and purified by silica gel chromatography. This compound exhibited strong absorptions at 2950 (methylene) and 1740 (ester carbonyl) $cm^{-1}$ in the IR spectrum. The $^{13}$C-NMR spectrum demonstrated 19 carbons which were identified as four C-CH$_3$, one =C-CH$_3$, one O-CH$_3$, five CH$_2$, five -CH, one >C=CH and one C=O carbons. The molecular formula of IV was established as $C_{19}H_{34}O_3$ by mass spectrum (m/z, 310 (M$^+$)) and $^1$H and $^{13}$C-NMR spectra data and the structure was determined as shown in Fig. 5 by the $^1$H-$^1$H, $^{13}$C-$^1$H COSY and $^{13}$C-$^1$H long range COSY spectral analyses.

## Antitumor Activity of BU-3983T

BU-3983T was tested for in vitro cytotoxicity against murine and human cell lines and for in vivo antitumor activity in mice. B16-F10 (murine melanoma) and Moser (human colorectal carcinoma) cells were grown to the logarithmic phase in the enriched Eagle minimum essential medium supplemented with fetal calf serum (FCS, 10%) and kanamycin (60 $\mu$g/ml), and HCT-116 (human colon carcinoma) were in McCoy's 5A medium supplemented with FCS (10%), penicillin (100 U/ml) and streptomycin (100 $\mu$g/ml). B16-F10, Moser and HCT-116 cells were harvested and implanted into wells of a 96-well microtiter plate at the inoculum sizes of 1.5 x $10^4$, 3 x $10^4$ and 3 x $10^4$ cells/ml, respectively. They were incubated with test materials at 37° C in humidified atmosphere of 5% $CO_2$ and 95% air for 72 hours. The cytotoxicity against tumor cells was determined colorimetrically at 540 nm after staining viable cells. The results are summarized in Table 5. Among the above 3 tumor cell lines, BU-3983T showed relatively specific cytotoxicity against B16-F10 cells with $IC_{50}$ value of 0.004 $\mu$g/ml, which was 100 and 10 times more potent than those against Moser and HCT-116 cells, respectively.

Inhibitory effects of BU-3983T on the macromolecule (DNA, RNA and protein) synthesis were determined in cultured B16-F10 melanoma cells. B16-F10 cells ($10^5$ cells/ml) were incubated with test materials at 37° C for 4.5 hours (for DNA synthesis) or 4 hours (for RNA and protein syntheses). Labelled precursor, $^3$H-thymidine, $^{14}$C-uridine or $^3$H-leucine was added to the culture and further incubated for 30 minutes (for DNA) or 60 minutes (for RNA and protein). After washing with chilled 5% trichloroacetic acid solution, the radioactivity incorporated into the acid-insoluble fraction of the tumor cells was determined by a liquid scintillation counter. Actinomycin D was used as a reference compound. As shown in Table 6, BU-3983T significantly inhibited both DNA and RNA syntheses with $IC_{50}$ values of 0.26 and 0.29 $\mu$g/ml, respectively, whereas it showed only weak inhibition of protein synthesis at 1.0 $\mu$g/ml.

## Table 3.  Physico-chemical properties of BU-3983T

Nature : White amorphous powder

M.P. : 155 - 158°C

$[\alpha]_D^{26}$ : -36.6° ± 0.1 (C 1.0, MeOH)

Elemental analysis : Calcd. for $C_{43}H_{73}N_7O_{13} \cdot H_2O$:

        C, 56.50%; H, 8.27%; N, 10.73%

        Found:  C, 56.74%; H, 8.31%; N, 10.78%

HR FAB MS (M-H)$^-$ : m/z 894.5217 (Calcd for $C_{43}H_{72}N_7O_{13}$  894.5188)

IR $\nu_{max}^{KBr}$ cm$^{-1}$ : 3380, 2960, 2930, 2870, 1750, 1650, 1460, 1410, 1240, 1105, 1000, 560

$^1$H-NMR (400 MHz in $CDCl_3$) : Fig. 3

$^{13}$C-NMR (100 MHz in $CDCl_3$) : Fig. 4

Rt in HPLC (min) : 9.2(18%), 11.4(82%)  Fig. 1

TLC ($SiO_2$, Merck $F_{254}$) :

    $CH_2Cl_2$-MeOH (9:1),    Rf 0.42, 0.54$^{trace}$

    n-BuOH-AcOH-$H_2O$ (3:1:1),  Rf 0.40, 0.53$^{trace}$

    (These spots are interchangeable on TLC plate)

Table 4.  $^1$H-NMR and  $^{13}$C-NMR spectra of lipophilic fragment (IV)

| Position No. | $^1$H-NMR (400 MHz, in CDCl₃) | $^{13}$C-NMR (100 MHz, in CDCl₃) |
|---|---|---|
| 1 | - | 171.2 (s) |
| 2 | 2.47 (m) / 2.67 (m) | 26.5 (t) |
| 3 | 1.92 (m) | 22.5 (t) |
| 4 | 3.48 (dd, 5.5 & 9.8) | 73.8 (d) |
| 5 | 4.63 (d, 5.5) | 86.2 (d) |
| 6 | - | 128.7 (s) |
| 7 | 5.22 (dt, 9.8 & 1.3) | 137.0 (d) |
| 8 | 2.52 (m) | 29.8 (d) |
| 9 | 1.0 - 1.3 (m) | 45.7 (t) |
| 10 | 1.43 (m) | 27.9 (d) |
| 11 | 1.0 - 1.3 (m) | 44.9 (t) |
| 12 | 1.37 (m) | 31.6 (d) |
| 13 | 1.0 - 1.3 (m) | 30.3 (t) |
| 14 | 0.85 (t, 7.5) | 11.4 (q) |
| 15 | 0.80 (d, 6.4) [a] | 19.0 (q) [b] |
| 16 | 0.78 (d, 6.4) [a] | 19.3 (q) [b] |
| 17 | 0.92 (d, 6.7) | 21.2 (q) |
| 18 | 1.67 (d, 1.3) | 12.5 (q) |
| 19 | 3.36 (s) | 56.8 (q) |

a) and b), Assignment may be interchanged

In vivo antitumor activity of BU-3983T was examined using tumor-bearing mice. Male BDF₁ mice were intraperitoneally inoculated with 0.5 ml of 10% melanotic melanoma B16 brei, and female CDF₁ mice were intraperitoneally inoculated with 0.4 ml of diluted ascitic fluid containing 10⁶ lymphocytic leukemia P388 cells or 10⁵ lymphoid leukemia L1210 cells. Mitomycin C was used as a reference compound. Test compounds were intraperitoneally administered to the mice by the following treatment schedules: once a day on day 1 only (Q1D x 1), on days 1, 5 and 9 (Q4D x 3) or on days 1 to 9 (Q1D x 9). BU-3983T showed moderate therapeutic activity against B16 melanoma with maximum T/C of 146-162 % by various treatment schedules (Table 7). In particular, the compound exhibited good antitumor activity with the best chemotherapeutic ratio by a Q1D x 1 treatment schedule. The compound showed no significant prolongation of lifespan in both P388 and L1210 leukemia systems (Tables 8 and 9).

7

Table 5

| In vitro cytotoxicity against murine and human tumor cells | | | |
|---|---|---|---|
| | $IC_{50}$ ($\mu$g/ml) | | |
| Compound | B16-F10 | Moser | HCT-116 |
| BU-3983T | 0.004 | 0.4 | 0.04 |

Table 6

| Inhibition of macromolecule synthesis in B16-F10 melanoma cells | | | |
|---|---|---|---|
| | $IC_{50}$ ($\mu$g/ml) | | |
| Compound | DNA | RNA | Protein |
| BU-3983T | 0.26 | 0.29 | >1.0 |
| Actinomycin D | 1.3 | 0.07 | >30 |

EP 0 431 323 A1

Table 7

| Antitumor activity of BU-3983T against B16 melanoma (ip) | | | | | |
|---|---|---|---|---|---|
| Compound | Dose (mg/kg/day) | Treatment schedule (ip) | MST[1] (day) | T/C (%) | Body weight change on day 5 (g) |
| BU-3983T | 8 | Q1D x 1 | 17.5 | 135[2] | -5.3 |
| | 4 | Q1D x 1 | 19.0 | 146[2] | -5.3 |
| | 2 | Q1D x 1 | 21.0 | 162[2] | -4.8 |
| | 1 | Q1D x 1 | 16.5 | 127[2] | -4.0 |
| | 0.5 | Q1D x 1 | 17.0 | 131[2] | -1.0 |
| | 0.25 | Q1D x 1 | 17.0 | 131[2] | 0.0 |
| | 0.13 | Q1D x 1 | 14.0 | 108 | +0.5 |
| BU-3983T | 2 | Q4D x 3 | 18.0 | 138[2] | -4.0 |
| | 1 | Q4D x 3 | 17.5 | 135[2] | -2.3 |
| | 0.5 | Q4D x 3 | 19.0 | 146[2] | -0.5 |
| | 0.25 | Q4D x 3 | 16.0 | 123 | 0.0 |
| | 0.13 | Q4D x 3 | 15.0 | 115 | +0.0 |
| | 0.063 | Q4D x 3 | 14.0 | 108 | +0.5 |
| Mitomycin C | 2 | Q4D x 3 | 27.5 | 212[2] | +0.5 |
| | 1 | Q4D x 3 | 22.0 | 169[2] | +0.8 |
| | 0.5 | Q4D x 3 | 18.0 | 138[2] | +0.6 |
| | 0.25 | Q4D x 3 | 15.5 | 119 | +0.8 |
| Vehicle | - | Q4D x 3 | 13.0 | - | +0.8 |
| BU-3983T | 1 | Q1D x 9 | Tox | Tox | - |
| | 0.5 | Q1D x 9 | 20.0 | 148[2] | -4.0 |
| | 0.25 | Q1D x 9 | 19.5 | 138[2] | -1.0 |
| | 0.13 | Q1D x 9 | 19.0 | 131[2] | -0.3 |
| | 0.063 | Q1D x 9 | 17.5 | 121 | +1.0 |
| | 0.031 | Q1D x 9 | 16.0 | 110 | +1.0 |
| Vehicle | - | Q1D x 9 | 14.5 | - | +0.5 |

* 1 Median survival time
* 2 Asterisk indicated significant antitumor effect (T/C $\geq$ 125%)

9

Table 8

| Antitumor activity of BU-3983T against P388 leukemia (ip) | | | | | |
|---|---|---|---|---|---|
| Compound | Dose (mg/kg/day) | Treatment schedule (ip) | MST[*1] (day) | T/C (%) | Body weight change on day 4 (g) |
| BU-3983 T | 8 | Q1D x 1 | 11.5 | 115 | -2.5 |
| | 4 | Q1D x 1 | 11.0 | 110 | -2.3 |
| | 2 | Q1D x 1 | 10.0 | 100 | -2.0 |
| | 1 | Q1D x 1 | 10.0 | 100 | -1.5 |
| | 0.5 | Q1D x 1 | 10.0 | 100 | -0.5 |
| | 0.25 | Q1D x 1 | 11.0 | 110 | +0.3 |
| Mitomycin C | 4 | Q1D x 1 | 16.5 | 165[*2] | -0.8 |
| | 2 | Q1D x 1 | 15.5 | 155[*2] | +0.5 |
| | 1 | Q1D x 1 | 14.0 | 140[*2] | +2.0 |
| | 0.5 | Q1D x 1 | 13.0 | 130[*2] | +1.5 |
| | 0.25 | Q1D x 1 | 11.5 | 115 | +1.0 |
| Vehicle | - | Q1D x 1 | 10.0 | - | +1.0 |

*1 Median survival time
*2 Asterisk indicates significant antitumor effect (T/C ≧ 125%)

Table 9

| Antitumor activity of BU-3983T against L1210 leukemia (ip) | | | | | |
|---|---|---|---|---|---|
| Compound | Dose (mg/kg/day) | Treatment schedule (ip) | MST[*1] (day) | T/C (%) | Body weight change on day 4 (g) |
| BU-3983 T | 8 | Q1D x 1 | Tox | Tox | - |
| | 4 | Q1D x 1 | 9.5 | 119 | -2.5 |
| | 2 | Q1D x 1 | 9.0 | 113 | -2.0 |
| | 1 | Q1D x 1 | 8.0 | 100 | -2.0 |
| | 0.5 | Q1D x 1 | 8.0 | 100 | -0.5 |
| | 0.25 | Q1D x 1 | 8.0 | 100 | +0.8 |
| Mitomycin C | 8 | Q1D x 1 | 12.5 | 156[*2] | -0.3 |
| | 4 | Q1D x 1 | 11.0 | 138[*2] | +0.5 |
| | 2 | Q1D x 1 | 10.5 | 131[*2] | +1.0 |
| | 1 | Q1D x 1 | 10.0 | 125[*2] | +1.5 |
| | 0.5 | Q1D x 1 | 10.0 | 125[*2] | +1.5 |
| Vehicle | - | Q1D x 1 | 8.0 | - | +1.6 |

*1 Median survival time
*2 Asterisk indicates significant antitumor effect (T/C ≧ 125%)

As indicated above BU-3983T possesses inhibitory activity against mammalian malignant tumors.

According to one aspect of the invention, therefore, there is provided a method for therapeutically treating a mammalian host affected by a malignant tumor sensitive to BU3983T which comprises administering to said host an effective tumor-inhibiting dose of BU-3983T.

In yet another aspect of this invention a pharmaceutical composition is provided which comprises an effective tumor-inhibiting amount of BU-3983T in combination with an inert pharmaceutically acceptable carrier or diluent. These compositions can be made up in any pharmaceutical form appropriate for the desired route of administration.

The pharmaceutical compositions provided by the present invention may contain other active ingredients, e.g. other antitumor or antimicrobial agents, and may be made up in any form appropriate for the desired route of administration. Examples of such compositions include solid compositions for oral administration such as capsules, tablets, pills, powders and granules, liquid compositions for oral administration such as solutions, suspensions, syrups or elixirs and preparations for parenteral administration such as sterile solutions, suspensions or emulsions. They may also be manufactured in the form of sterile solid compositions which can be dissolved in sterile water, physiological saline or other sterile injectable medium immediately before use.

It will be appreciated that the actual preferred dosages of the compounds of the present invention will vary according to the particular compound being used, the particular composition formulated, the mode of administration and the particular situs, host and disease being treated. Many factors that modify the action of the drug will be taken into account by those skilled in the art, e.g. age, body weight, sex, diet, time of administration, route of administration, rate of excretion, condition of the hose, drug combinations, reaction sensitivities and severity of the disease. Optimal dosages for a given set of conditions can be ascertained by those skilled in the art using conventional dosage determination tests in view of the experimental animal data provided.

While the invention has been described with respect to various specified examples and embodiments, it is to be understood that the invention is not limited thereto.

Example 1
Fermentation of BU-3983T

A loopful of the mature slant culture of Actinomadura verrucosospora strain No. Q886-2 was inoculated into a 500-ml Erlenmeyer flask containing 100 ml of the seed medium consisting of 2% glycerol, 1% soybean meal and 0.5% of $CaCO_3$ (pH 7.0). The seed culture was incubated at $32\,^\circ C$ for 4 days on a rotary shaker (200 rpm) and 5 ml portion of the seed culture was transferred into a 500-ml Erlenmeyer flask containing 100 ml of the fermentation medium having the same composition as the seed medium. Fermentation was carried out at $28\,^\circ C$ for 6 days on a rotary shaker (200 rpm).

The scale up fermentation was carried out in a 20-liter stir jar fermentor. Five hundred milliliters of the seed culture prepared by flask fermentation was inoculated into a 20-liter stir jar fermentor containing 12 liters of the fermentation medium. The jar fermentor was operated at $32\,^\circ C$ with agitation of 250 rpm and aeration rate of 12 liters per minute. The antibiotic production was monitored by the in vitro cytotoxic activity against B16 melanoma cell, the fermented broth after 90-hour cultivation showed the activity at x256 dilution.

Example 2
Isolation and purification

The whole cultured broth (26L, pH 7.3) was stirred with n-butanol (15L) for 1 hour. The organic layer was separated by a Sharples-type centrifuge (Kokusan No. 4A), and concentrated to dryness under reduced pressure. The residue (54 g) was applied on a column of silica gel (Wako gel C-200, $\Phi$ 4.0 x 50 cm) previously equilibrated with methylene chloride, and developed with methylene chloride-methanol solution (stepwise increase of methanol from 100:0 to 90:10, v/v). The fractions were monitored by cytotoxicity against B16 melanoma and color reaction with ammonium molybdate-sulfuric acid (AMS) on TLC plate. Active eluates were combined, concentrated and charged on a column of silica gel ( $\Phi$ 3.5 x 5.0 cm). Elution was performed by ethyl acetate-methanol (50:1) and the pooled active eluates were concentrated. The residue was further purified by Sephadex LH-20 chromatography with methanol elution. Active fractions were combined, evaporated in vacuo and lyophilized to yield a pure solid of BU-3983T (518 mg).

Example 3
Degradation of BU-3983T
1. Complete acid hydrolysis

BU-3983T (300 mg) dissolved in 6N HCl (15 ml) was heated in a sealed tube for 16 hours at $105\,^\circ C$. After addition of water (90 ml), the reaction mixture was extracted with ethyl ether (50 ml, x 2). Evaporation of the ethyl ether extract afforded an oily residue (90 mg) containing unidentified lipophilic fragments. The aqueous layer was concentrated in vacuo to a sticky solid (240 mg) which was applied on a column of Dowex 50W x 4 (pyridine form, $\phi$ 1.5 x 75 cm). The column was developed with 0.1M pyridine-formic acid (pH 3.1) and 0.2M pyridine-formic acid (pH 3.1) successively and the eluate was monitored by TLC (n-BuOH-AcOH-$H_2O$, 63:10:27, ninhydrin detection). Glycine(I) and sarcosine(II) were eluted with 0.1M pyridine-formic acid, while an unidentified amino acid (III) was with 0.2M pyridine-formic acid. The fractions containing III were pooled, evaporated and desalted with Sephadex LH-20 chromatography developed with 50% aqueous methanol to yield a pure solid of amino acid III (6.4 mg).

III: white amorphous solid; SIMS m/z 148(M + H)$^+$, 129(M-$H_2O$)$^+$;

$^1$H-NMR(400 MHz, $D_2O$) $\delta$ 1.03(3H,d,J = 6.6Hz), 1.04(3H,d,J = 6.6Hz) 2.05(1H,m), 3.59(1H,dd,J = 2.9 & 10.5Hz), 4.28(1H,d,J = 2.9Hz);

$^{13}$C-NMR (100 MHz, $D_2O$) $\delta$ 19.3 ( q), 19.4 ( q), 31.1(d), 58.0(d), 96.9(d), 172.6(s)

## 2. Alkaline hydrolysis

A solution of BU-3983T (300 mg) in 1N NaOH (15 ml) was refluxed for 1.5 hours at 105°C. The reaction mixture was neutralized with 1N HCl (15 ml), diluted to 100 ml with water and extracted with ethyl ether (100 ml x 3). Ethyl ether layer was combined, dried over $Na_2SO_4$ and evaporated in vacuo to yield yellow oil (67 mg). It was charged on a column of silica gel which was developed with methylene chloride to afford the lipophilic fragment (IV).

IV: colorless oil; EIMS m/z 310(M$^+$);

$$IR \ \nu_{max}^{KBr}$$

cm$^{-1}$ 3460, 2950, 2930, 1740, 1460, 1380, 1105; $^1$H and $^{13}$C-NMR in Table 4

## Claims

1. The antitumor antibiotic compound BU-3983T which has the following characteristics:
   (a) soluble in acetonitrile, methanol, chloroform dimethyl sulfoxide an dimethylformamide, slightly soluble in n-butanol, ethanol and ethyl acetate, but practically insoluble in n-hexane and water;
   (b) gives a positive response to iodine vapor, AMS, and Rydon-Smith reagents;
   (c) it is negative to ninhydrin, anthrone and Sakaguchi reaction;
   (d) is an effective antitumor agent against B16 melanoma;
   (e) acid hydrolysis with 6N hydrochloric acid produces glycine, sarcosine, $\beta$-hydroxyleucine along with an unseparable mixture of lipophilic fragments;
   (f) has HPLC profile substantially as shown in FIG. 1;
   (g) has an infrared absorption spectrum substantially as shown in FIG. 2;
   (h) has an $^1$H-NMR absorption spectrum substantially as shown in FIG. 3;
   (i) has an $^{13}$C-NMR absorption spectrum substantially as shown in FIG. 4;
   (j) shows the degradation products substantially as shown in FIG. 5.
   (k) has molecular formula: $C_{43}H_{73}N_7O_{13}$

2. A process for producing BU-3983T which comprises cultivating a BU-3983T-producing strain of Actinomadura verrucosospora under submerged aerobic conditions in an aqueous nutrient medium containing assimilable sources of carbon and nitrogen until a substantial amount of BU-3983T is produced by said organism in said culture medium and then recovering the BU-3983T from the culture medium.

3. The process according the Claim 2 wherein the BU-3983T-producing strain is Actinomadura verrucosospora ATCC-53905 or a mutant or variant thereof.

4. A biologically pure culture of the microorganism Actinomadura verrucosospora ATCC-53905 capable of producing the antibiotic BU-3983T upon cultivation in an aqueous nutrient medium containing assimilable sources of carbon and under submerged aerobic conditions, or a variant or mutant of Actinomadura verrucosospora ATCC-53905 which retains the characteristic of BU-3983T production.

5. A pharmaceutical composition comprising a tumor-inhibiting amount of BU-3983T as defined in claim 1 and a pharmaceutical carrier.

6. A process for preparing the pharmaceutical composition of claim 5 which comprises incorporating BU-3983T as defined in claim 1 into a pharmaceutical carrier.

7. The use of BU-3983T as defined in Claim 1 for preparing a pharmaceutical composition for therapeutically treating an mammalian host affected by a tumor.

Fig. 1.   HPLC profile of BU-3983T

## Conditions

| | |
|---|---|
| Column | : M & S pack C18 (M & S Instruments) |
| Solvent | : $CH_3CN-H_2O$ (75:25) |
| Flow rate | : 1 ml/min |
| Detection | : UV 215 nm |

Fig. 3. $^1$H-NMR spectrum of BU-3983T in CDCl$_3$

Fig. 4. $^{13}$C-NMR spectrum of BU-3983T in CDCl$_3$

17

Fig. 5    Degradation products of BU-3983T

CH₂COOH
|
NH₂                                    Glycine (I)

CH₂COOH
|
NHCH₃                                  Sarcosine (II)

CH₃
  \
    CH-CH-CH-COOH                      β-hydroxyleucine (III)
  /      |   |
CH₃     OH  NH₂

Lipophilic fragment (IV)

European Patent Office

# EUROPEAN SEARCH REPORT

Application Number

**EP 90 12 1149**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | THE JOURNAL OF ANTIBIOTICS, vol. 38, no. 11, 1985, pages 1605-1609; M. KONISHI et al.: "Esperamicins, a novel class of potent antitumor antibiotics. I. Physico-chemical data and partial structure" <br> * Complete article * <br> — — — | 1-2,5 | C 12 P 1/06 <br> C 12 N 1/20 <br> C 07 G 11/00 <br> A 61 K 35/66 // <br> (C <br> 12 P 1/06 |
| Y | DE-A-3 418 023 (BRISTOL) <br> * Completely * <br> — — — | 1-2,5 | C 12 R 1:03 ) <br> (C 12 N 1/20 <br> C 12 R 1:03 |
| A | US-A-4 195 079 (CELMER) <br> * Completely * <br> — — — | 1,5 | ) |
| A | EP-A-0 305 086 (ELI LILLY) <br> * Completely * <br> — — — | 1,5 | |
| A | GB-A-2 179 649 (BRISTOL) <br> * Completely * <br> — — — — — | 1,5 | |

| TECHNICAL FIELDS SEARCHED (Int. Cl.5) |
|---|
| C 12 P <br> C 12 N <br> C 12 R <br> A 61 K |

The present search report has been drawn up for all claims

| Place of search | Date of completion of search | Examiner |
|---|---|---|
| The Hague | 15 February 91 | RAJIC M. |